# EUROPEAN PATENT APPLICATION

(11) **EP 0 937 776 A2**
(43) Date of publication of application: **25.08.1999**
(21) Application number: 99103208.7
(22) Date of filing: 18.02.1999
(51) Int. Cl.: C12N 11/02, C12N 11/08, C12N 11/14, C12N 11/18

(54) **Immobilized enzymes**

(30) Priority: 18.02.1998 JP 3594098; 02.09.1998 JP 24862298
(71) Applicant: Kabushiki Kaisha Toyota Chuo Kenkyusho, Aichi-gun, Aichi-ken, 480-1192 (JP)
(72) Inventor: Takahashi, Haruo c/o K.K. Toyota Chuo Kenkyusho, Aichi-gun, Aichi-ken, 480-1192 (JP); Inagaki, Shinji c/o K.K. Toyota Chuo Kenkyusho, Aichi-gun, Aichi-ken, 480-1192 (JP); Kajino, Tsutomu c/o K.K. Toyota Chuo Kenkyusho, Aichi-gun, Aichi-ken, 480-1192 (JP); Usuki, Arimitsu c/o K.K. Toyota Chuo Kenkyusho, Aichi-gun, Aichi-ken, 480-1192 (JP); Li, Bo c/o K.K. Toyota Chuo Kenkyusho, Aichi-gun, Aichi-ken, 480-1192 (JP)
(74) Representative: Blumbach, Kramer & Partner GbR

(57) **Abstract**

The invention features an immobilized enzyme wherein an enzyme or an active unit of the enzyme is immobilized in a structural unit having structural stability; namely, the present invention provides an immobilized enzyme comprising: a structural unit having structural stability; and an enzyme having an active unit, the enzyme, or the active unit being immobilized in the structural unit; in the preferred aspects, the structural unit is a porous substance having homogeneous pores, a pore size (diameter) of the porous substance of the structural unit is almost the same as that of the enzyme to be immobilized or the active unit of the enzyme, and the structural unit comprises a mesoporous silica porous material to be formed via a layered silicate.

## Description

### BACKGROUND OF INVENTION

### 1. Field of the Invention

The present invention relates to an immobilized enzyme with advantages of an enzyme and those of an inorganic catalyst.

### 2. Related Arts

Enzymes have advantages such as exhibition of catalytic activity at normal temperature, high specificity of catalysis and less side-reaction, but have drawback such as comparatively low stability. On the other hand, inorganic catalysts have advantages such as high stability and versatility of reactions, but have such drawbacks that the reaction retires high energy such as high temperature or high pressure, that the specificity of the reaction is low and that the side-reaction is liable to arise. Accordingly, there has never been known a catalyst with advantages of enzymes and that of the inorganic catalysts, such as high reactivity at normal temperature, high specific reactivity and less side-reaction.

Enzymes are formed from polypeptides comprising amino acids bonded each other being folded in a certain form to form a stereostructure in which an active site is formed. A mechanism wherein the enzyme having such a structure is inactivated includes the case where a polypeptide chain constituting the enzyme is cleaved by a protease and the case where the conformation of a protein changes with a change of the eternal environment such as pH and the active site is broken. As a method of preventing inactivation of these enzymes, a trial of newly introducing crosslinking into a protein molecule by using a S-S bond and glutaraldehyde, thereby to make the structure of the protein molecule itself rigid has been made. Such a modification, however, should vary dependent on particular enzymes and sufficient stability is not obtained sometimes. Therefore, it is inferior in utility.

As a method to be applied for stabilization of various enzymes, for example, a method using an immobilized enzyme (Ichiro CHIBATA et al., Immobilized Biocatalyst, Kodansha Scientific) has been suggested.

In a conventional immobilized enzyme, since a protein is directly immobilized in a resin, the protein is decomposed by a protease and it is impossible to prevent the stereostructure from changing with a change of the external environment. Furthermore, according to these methods wherein an inclusive immobilization method for entrapment of an enzyme in a gel and a microcapsule method of coating it with a semitransparent coat are suggested, the enzyme is not decomposed by the protease and an improvement in stability is expected.

In these methods, however, since the enzyme and the structural material for coating the exterior portion are not generally immobilized in the form of agreeing with a molecular size, the enzyme can not be firmly immobilized in a gel lattice or a capsule, resulting in defects such as leakage and inactivation of the enzyme. Moreover, it is difficult to prevent the conformational change thereof by the external environment.

On the other hand, a method of stabilizing an enzyme by modifying the surface of a protein using polyethylene glycol (INADA et al., Unexamined Patent Publication (Kokai) No. 2-222698) and glycolipid (OKAHATA et al., Journal of Organic Chemistry, Vol. 60, page 2244 (1995)) has been suggested. However, since the structural material for coating the enzyme according to these methods does not match to the molecular size and the structural stability is insufficient, it is difficult to prevent a conformational change of the enzyme with the external environment.

On the other hand, a so-called artificial enzyme has been suggested and, for example, there have been known one wherein enzyme activity is produced by bonding metal phthalocyanaine with a polymer substance (Examined Patent Publication (Kokoku) No. 2-5765/Unexamined Patent Publication (Kokai) No. 2-57260) and one wherein a catalysis is intended to be enhanced by introducing an imidazole group into porphyrin and chelating it (Science, 275, 949-951, 1997). According to these means, the stability is improved but the specificity is by far inferior to a natural enzyme.

### SUMMARY OF THE INVENTION

Accordingly, an object of the present invention is to provide an immobilized enzyme with advantages of an enzyme and that of an inorganic catalyst, such as exhibition of catalytic action at normal temperature, long-term stability, high reaction specificity and less secondary reaction. The present inventors have found in the present invention that a porous substance having a pore size close to a diameter of an enzyme molecule regardless of the kind of an enzyme to be immobilized is most excellent in immobilization ratio and enzyme stabilization capability. Thus, the present invention has been accomplished.

That is, the present invention provides an immobilized enzyme comprising:
a structural unit having structural stability; and
an enzyme having an active unit, the enzyme, or the active unit being immobilized in the structural unit In the above structural unit, one or more enzyme molecules are preferably contained while they match with the pore size. Preferable structural unit includes, for example, mesoporous material and vinyl polymer.

In an aspect of the present invention, the structural unit is a porous substance having homogeneous pores.

In another aspect of the present invention, a pore size (diameter) of the porous substance of the structural unit is larger than 2 nm.

In another aspect of the present invention, a pore size (diameter) of the porous substance of the structural unit is almost the same as that of the enzyme to be immobilized or the active unit of the enzyme.

In another aspect of the present invention, the silicon-containing compound is a mesoporous silica porous material to be formed via a layered silicate.

In another aspect of the present invention, the mesoporous silica porous material is FSM.

In another aspect of the present invention, the surface of the structural unit is anionic.

In another aspect of the present invention, the structural unit further comprises an anchor unit for immobilization of the enzyme or the active unit of the enzyme in the structural unit.

In another aspect of the present invention, the pore size (diameter) of the porous substance of the structural unit is smaller than 30 nm.

In a further aspect of the present invention, the pore size (diameter) of the porous substance of the structural unit is smaller than 10 nm.

In a further aspect of the present invention, the enzyme is at least one selected from the group consisting of oxidase, reductase, hydrolase, lipase and isomerase.

In a still more aspect of the present invention, the enzyme is at least one selected from-the group consisting of peroxidase, subtilisin and lipase.

In the present invention, an enzyme molecule or an active unit of an enzyme is coated with a structurally stable structural unit in the form of matching with the shape of the molecule. Therefore, an enzyme or its active unit is not subjected to an action of a protease. In addition, since the enzyme or its active unit is put in the structural unit in the form of matching with the shape of the molecule, it is possible to effectively prevent the degree of freedom of the stereostructure of the active unit from being limited and to effectively prevent the stereostructure of the active unit from changing with a change of the external environment.

That is, the present invention has a feature which is not found in the concept of conventional immobilized enzymes, for example, stability of physical properties such as heat and pH as well as chemical stability such as stability in an organic solvent remarkably superior to conventional enzymes such as immobilized enzymes, polyethylene glycol (PEG) modified enzymes and lipid-modified enzymes.

### BRIEF DESCRIPTION OF THE DRAWINGS

Fig. 1 is a general schematic diagram showing the structure of the immobilized enzyme according to the present invention.
Fig. 2 is a schematic diagram showing the structure of the immobilized enzyme immobilized in FSM.

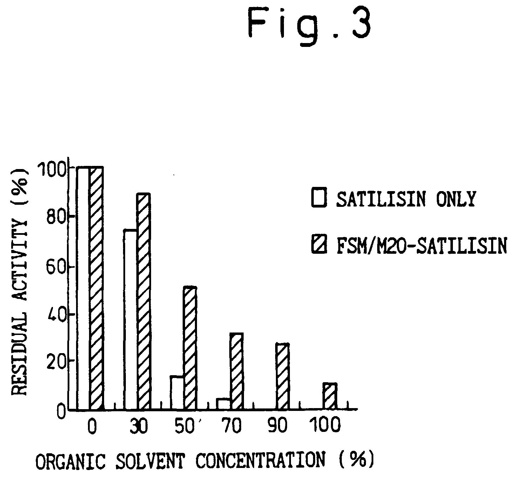

Fig. 3 is a graph showing the organic solvent resistance of subtilisin alone or FSM/M20-subtilisin.
Fig. 4 is a graph showing the reaction process in case where an enzyme reaction is performed in toluene using various porous materials in which peroxidase is immobilized.
Fig. 5 is a graph showing the reaction process in case where an enzyme reaction is performed in toluene using various porous materials in which peroxidase is immobilized.
Fig. 6 is a graph showing the amount of various surfactants adsorbed to the porous material according to the present invention.

### [DESCRIPTION OF REFERENCE NUMERALS]

- 1: structural unit
- 2: enzyme or its active unit
- 3: anchor unit
- 4: substrate

### EMBODIMENT OF CARRYING OUT THE INVENTION

The present invention can be applied to an environment-suitable type industrial process which has not conventionally been realized because of unstableness of the enzyme. For example, the immobilized enzyme of the present invention can be used in biosensor, biobleaching, biodesulfurization, biomass and degradation of environmental pollutant.

Furthermore, in the present invention, it is not necessary to bond an enzyme with an immobilization carrier by the chemical reaction, like a conventional immobilized enzyme, and the immobilized enzyme can be easily prepared by mixing them in water or a suitable buffer. Accordingly, the enzyme or its active unit is hardly inactivated during the immobilization, like a conventional method. Moreover, since the pore size matches with the size of the molecule, the resulting immobilized enzyme is superior in stability over a conventional immobilized enzyme.

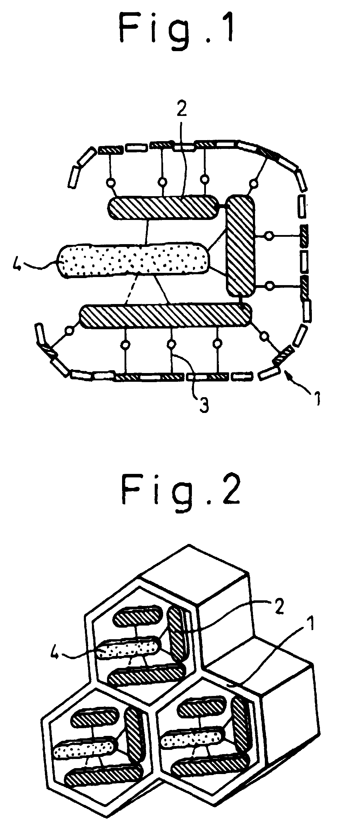

One example of the structure of this immobilized enzyme can be schematically shown as in Fig. 1. In this drawing, 1 denotes a structural unit, which has resistance to the environmental conditions such as pH and heat and can retain the shape. An enzyme or its active unit 2 is a portion capable of producing an enzyme activity by an interaction with a substrate 4, and is composed of a native enzyme or a fragment of an enzyme containing an active site.

In addition, an anchor unit 3 is an element for connecting the structural unit 3 to the enzyme or its active unit 2, which communicates environmental stability of the structural unit to the enzyme or its active unit, thereby to control a large structural change of the enzyme or its active unit and to maintain the enzyme or its active unit stable, and which adds the degree of freedom enough to permit the structural change in case where the active unit changes the stereostructure by an interaction with the substrate. However, the anchor unit is not an essential constituent element, necessarily.

In Fig. 2, another structure of the immobilized enzyme according to the present invention is shown, and an enzyme or its active unit and a structural unit are bonded by a van der Walls force without using an anchor unit.

Each structural unit contains one or more enzyme molecules or their active units. Accordingly, it is necessary that pores of the structural unit has a suitable size enough to contain the enzymes or their active units, and suitable size varies depending on the size of the enzyme or its active unit. The structural unit may also be made of an inorganic material or an organic material, particularly polymer.

The organic material may be any one wherein a polymer material can be obtained by polymerizing a monomer, and examples thereof include polymers obtained from a monofunctional monomer having a group such as double bond, capable of polymerizing by means of heat and light, or a polyfunctional monomer having two or more groups.

Specific examples of the monomer include acrylic acid, methyl(meth)acrylic acid, ethyl(meth)acrylic acid, methyl (meth)acrylate, ethyl (meth)acrylate, hydroxyethyl (meth)acrylate, hydroxypropyl (meth)acrylate, styrene, divinylbenzene, acrylamide and 4-vinylpyridine, but are not limited thereto. Particularly preferred monomer is a vinyl monomer. These monomers can be used alone or in combination thereof.

To coat around the enzyme or its active unit, if desired via the anchor unit, a polymer formation reaction is required. The monomer material required for this reaction may be any monomer wherein a polymer material can be obtained by polymerizing the monomer, and examples thereof include a monofunctional monomer having a group such as double bond, capable of polymerizing by means of heat and light, or a polyfunctional monomer having two or more groups.

Specific examples thereof include polyacrylic acid, polymethyl(meth)acrylic acid, polyethyl(meth)acrylic acid, polymethyl (meth)acrylate, polyethyl (meth)acrylate, poly-2-hydroxyetnyl (meth)acrylate, poly-1- or poly-2-hydroxyethyl (meth)acrylate, polystyrene, polydivinylbenzene, and Kayarad R-167 and Kayarad PET-128 (these two products are manufactured by Nippon Kayaku Co.).

The inorganic materials are metal oxides such as SiO₂, Al₂O₃, TiO₂, ZrO₂ and Nb₂O₃ as well as a composite oxides such as that represented by SiO₂-MO_{2/n} (M is a metal such as Al). For example, when a structural unit having the composition of SiO₂ is formed, layered silicate such as kanemite, Si(OR)₄ (R is an alkyl group), silica gel, water glass and sodium silicate are used. Also in the case of the composition of the metal oxides other than SiO₂, alkoxide, oxide and salt, which contain the corresponding metal, are used. Dependent on the kind of the inorganic starting material, optimum pH of the reaction mixture and kind of the surfactant are selected.

To make a structural unit from the inorganic material, by mixing and reacting the inorganic starting material with the surfactant, there can be formed a surfactant/inorganic composite material wherein an inorganic skeleton is formed around micelles of the surfactant. When the surfactant is removed from this composite material, for example, by calcining at 400-600°C or extraction with an organic solvent, meso pores having the same shape as that of micelles of the surfactant are formed in the inorganic skeleton.

In the method for making the above structural unit, when a silicon-containing compound, e.g. silicon oxide such as SiO₂ is used as a starting material, pores can be formed by forming a layered silicate such as kanemite, interposing micelles between these layers, joining the space between layers, wherein micelles are not present, with a silicate molecule, and removing micelles. Alternatively, pores are formed by using a silicon-containing substance such as water glass as a starting material, gathering silicate monomers around micelles, polymerizing to form a silica, and removing micelle molecules. In this case, micelles are usually in the columnar form and, as a result, columnar pores are formed.

In any case, the structural unit is formed as a mesoporous molecular sieve having meso pores. In the former method to be performed through a layered silicate such as kanemite, the surface of pores is hydrophobic and anionic compared with the latter method to be performed by using water glass. The hydrophobic surface is preferable for stable immobilization of the non-hydrated enzyme or its active unit, and the anionic surface is preferable for immobilization of the enzyme having a cation on the surface or its active unit.

The micelles used for making the structural unit are famed by dispersing a surfactant in a suitable medium. As the surfactant, any of cationic, nonionic and anionic surfactants can be used. As the cationic surfactant, those having an ammonium group such as alkyltrimethylammonium is often used. As the nonionic surfactant, polyethylene glycol surfactants are used. As the anionic surfactant, alkyl sulfonate, alkyl phosphate and fatty salt are used, but are limited thereto.

By changing the length of the alkyl chain of the surfactant, the size of the micelles changes. As a result, the sizes of the pores to be formed can be controlled. By using comparatively hydrophobic molecules such as trimethylbenzene or tripropylbenzene in combination with a surfactant, the micelles are swollen, thereby making it possible to form pores with lager size. By using these methods, pores in the structural unit having an optimum size can be formed according to the size of the enzyme or its active unit.

When the inorganic starting material is mixed with a surfactant, suitable solvent may also be used. As the solvent, for example, water and alcohol are used.

The micelles to be formed by a surfactant in the structure includes, for example, spherical micelles, cylindrical micelles and layered micelles. A liquid crystal structure having a hexagonal or cubic structure can be formed by regularly arraying them.

The form of the formable structural unit includes, for example, powder, granule, sheet, bulk and film.

The pore size (diameter) of the porous substance constituting the structural unit is preferably almost the same as the diameter of the enzyme or the active unit of the enzyme, to be immobilized therein. This diameter is usually larger than 1 nm, and preferably larger than 2 nm. This pore size is generally smaller than 30 nm, and is usually smaller than 10 nm. According to the present invention, since the pore size can be selected from a wide range by selecting the surfactant capable of forming micelles, enzymes having various molecular weights or their active units can be stably immobilized.

Regarding the pore size distribution in the porous substance, the half-height width is preferably not more than 60% based on a mean pore size. Consequently, the size of the pores becomes uniform and, therefore, the enzyme or its active unit can be stably supported over whole pores if the pores are optimum to the enzyme or its active unit. As a result, the stability of the enzyme or its active unit is enhanced as possible.

The structural unit may have one or more peaks of the pore size distribution. When it has one peak of the pore size distribution, it is preferred to immobilize one enzyme or its active unit or two or more enzymes or their active units having almost the same molecular weights and/or particle sizes. When two or more peaks of the pore size distribution are present, it is preferred to two or more enzyme or their active units having the different molecular weights and/or particle sizes suited for the pore sizes.

In the case of immobilizing a plurality of enzymes or their active units in one structural unit, a plurality of enzymes or their active units can be supported in one structural unit at a predetermined quantitative proportion and a predetermined positional distribution by using a structural unit having a plurality of pore size distributions suited for each enzyme or its active unit, or using different anchor units suited for the enzyme or its active unit. As described above, by immobilizing a plurality of enzymes or their active units in the structural unit, the enzyme reaction involving a plurality of enzymes can be efficiently performed.

When the size of the pores sufficiently match to the size of the enzyme or its active unit to be introduced therein, since the major portion of the enzyme surface is in the proximity of the pore internal surface the enzyme or its active unit can be maintained in the pores by a van der Walls force.

According to another aspect, the enzyme or its active unit and the structural unit can also be bonded by means of an anchor unit between the enzyme or its active unit and the pore internal surface of the structural unit.

It is desired that molecule constituting the anchor unit preferably basically has the same structure as that of the structural unit. To bond to the active unit, it is particularly necessary to bond a functional group such as hydroxyl group, amide group, amino group, pyridine group, urea group, urethane group, carboxyl group, phenol group, azo group, hydroxyl group, silane group and aminoalkylene group.

The enzyme or its active unit may be not only a whole enzyme molecule, but also a fragment of an enzyme containing an active site. The enzyme or its active unit may be prepared by extracting from animals and plants and optionally cleaving the extract, or may also be synthesized by using a gene engineering or a chemical technique. The enzyme which can be used in the present invention includes, for example, oxidoreductases such as peroxidase; various transferases; hydrolases such as protease; various isomerases; and ligase.

To assemble the immobilized enzyme of the present invention, first, a polymerizing reaction is performed via a molecule to be connected directly to the enzyme or its active unit, thereby to form a structural unit so as to contain the enzyme or its active unit. Alternatively, first, the structural unit is formed and then the enzyme or its active unit may be introduced via an amino group of lysine of the enzyme or its active unit itself.

Bonding between the enzyme or its active unit and structural unit may be not only van der Waals bonding and covalent bonding, but also non-covalent bonding such as ionic bonding, hydrogen bonding and hydrophobic bonding.

### EXAMPLES

The following examples further illustrate the present invention in detail.

### Example 1. Structure 1 of mesoporous material (FSM/8, 10, 12, 14, 16, 18)

A powdered sodium silicate (SiO₂/Na₂O = 2.00) manufactured by Nippon Chemical Industries Co., Ltd. was calcined in an air at 700°C for 6 hours to form a crystal of sodium disilicate (delta-Na₂Si₂O₅). 50 g of this crystal was dispersed in 500 cc of water and stirred for 3 hours. Thereafter, the solid content was recovered by filtration to obtain a kanemite crystal as a layered silicate.

Without drying this kanemite, 50 g (dry basis) of kanemite was dispersed in 1000 ml of an aqueous 0.1 M solution of hexadecyltrimethylammonium chloride [C₁₆H₃₃N(CH₃)₃Cl] as a surfactant and then heated with stirring at 70°C for 3 hours. The pH of the dispersion at the initial stage of heating was 12.3. Then, the pH of the dispersion was lowered to 8.5 by adding 2N hydrochloric acid with heating/stirring at 70°C. Furthermore, the dispersion was heated at 70°C for 3 hours and air-cooled to room temperature. The solid product was once filtered, dispersed in 100 ml of deionized water and then stirred. This filtration/dispersion/stirring operation was repeated five times, followed by drying at 60°C for 24 hours. This sample was heated in a nitrogen gas at 450°C for 3 hours and then calcined in an air at 550°C for 6 hours to obtain a mesoporous material (FSM/16).

In the same operation as described above, a mesoporous material was synthesized by using octadecyltrimethylammonium chloride [C₁₈H₃₇N(CH₃)₃Cl] and the resulting mesoporous material was designated as FSM/18. Furthermore, six kinds of mesoporous materials were produced by using four kinds of alkyltrimethylammonium [CₙH₂ₙ₊₁N(CH₃)₃]chlorides (n = 14) or bromides (n = 8, 10, 12), which have different lengths (n) of a [CₙH₂ₙ₊₁] chain, in place of hexadecyltrimethylammonium chloride. The resulting mesoporous materials were respectively designated as FSM/8, FSM/10, FSM/12 and FSM/14, in which the numerical denotes a number (n) of the length of the alkyl chain length of the alkyltrimethylammonium used.

### Example 2. Structure 2 of mesoporous material (FSM/M05, 10, 20)

Under the same conditions as those of the method for production of the mesoporous material of Example 1, except that mesitylene [C₆H₃(CH₃)₃] was added in addition to 0.1 mol of hexadecyltrimethylammonium chloride, a mesoporous material was produced. The production was performed under three conditions of the amount of mesitylene to be added, that is, 0.05 mol, 0.1 mol and 0.2 mol. The resulting mesoporous materials were designated as FSM/M05, FSM/M10 and FSM/M20, respectively.

### Example 3

A method of immobilizing subtilisin as a kind of protease in pores of the mesoporous material FSM/M20 (pore diameter = 4.7 mm) shown in the production method of Example 2 will be explained. To 100 ml of deionized water in which 1.0 g of subtilisin (manufactured by Sigma Co.) was dissolved, 3 g of a powder of FSM/M20 was added and the solution was slowly stirred up and down at room temperature for about 5 hours. Then, the FSM/M20 powder was isolated by filtration and washed with deionised water. The powder was further dried at room temperature to obtain a mesoporous material powder in which subtilisin had been immobilized.

Thermogravimetric analysis (TG) of the mesoporous material powder in which subtilisin had been immobilized was carried out. As a result, two weight losses were observed within a range from room temperature to 150°C and a range from 200 to 500°C. The former weight loss is caused by elimination of adsorbed water and the latter is caused by thermal decomposition of subtilisin. The amount of subtilisin immobilized to the mesoporous material was calculated from the weight loss caused by thermal decomposition of subtilisin. As a result, it was about 10% based on the weight of the mesoporous material.

### Example 4

The FSM/M20-coated subtilisin (1 mg) obtained in Example 3 was added to 1 ml of a buffer solution (100 mM Tris-HCl, pH9.0) and incubation was performed at a predetermined temperature for 1 hour. After ice cooling, a 0.5% azocasein solution (manufactured by Sigma Co: 100 mM Tris-HCl, pH9.0) was added and the reaction was performed at 37°C for 1 hour. After the reaction was terminated by adding 2 ml of a 10% trichloroacetic acid, the reaction solution was allowed to stand at room temperature for 20 minutes and centrifuged. To this supernatant fraction, 0.5N NaOH was added and the activity of subtilisin was measured by the absorbance at 440 nm. A natural subtilisin as the comparative example lost almost all of the activity (<10%) after a heat treatment at 70°C for 1 hour, whereas, the FSM/M20-coated subtilisin maintained high activity (>80%) even after the heat treatment at 70°C for 1 hour.

### Example 5

1000 ml of a solution of subtilisin (concentration: 10 mg/ml) was mixed with 0.33 g of methacrylic acid, 5.0 g of 2-hydroxyethyl methacrylate, 0.2 g of a crosslinkable monomer (difunctional vinyl monomer) Kayarad R167 (Nippon Kayaku Co.), 0.1 g of a polymerization initiator azobisiobutyronitrile (AIBN) and 0.5 g of distearyldimethylammonium chloride, and the mixture was vigorously stirred to obtain a dispersion.

After allowing to stand overnight, the dispersion was polymerized with stirring at 60°C for 6 hours to produce a polymer-coated subtilisin.

### Example 6

The reaction solution obtained in Example 5 was added in a 10-fold amount of ethanol and a polymer-coated subtilisin was recovered by centrifugation. 1 mg of the polymer-coated subtilisin was added to a buffer solution (100 mM Tris-HCl, pH9.0) and incubation was performed at a predetermined temperature for 1 hour. After ice cooling, the residual activity of subtilisin was measured by the method described in Example 4. A natural subtilisin as the comparative example lost almost all of the activity (<10%) after a heat treatment at 70°C for 1 hour, whereas, the polymer-coated subtilisin maintained high activity (>80%) even after the heat treatment at 70°C for 1 hour.

### Example 7

Solutions (100 mg/ml) of cytochrome C and subtilisin (both are manufactured by Sigma Co.) as enzymes were respectively prepared by using deionized water. Various enzyme solution (5 ml) were added to various synthesized mesoporous materials (200 mg) and slowly mixed at 4°C for not less than 4 hours.

The supernatant was removed by centrifugation to recover the resulting porous material to which the enzyme had been bonded, and the porous material was washed three times. In this case, the amount of cytochrome C before bonding in the original enzyme solution, that of subtilisin in the supernatant to he bonded of, and wash after bonding were calculated from the absorbance at 403 nm (cytochrome C) and absorbance at 280 nm (subtilisin), respectively.

The results are summarized in Table 1. In the case of the porous material having a pore size of not more than 2 nm, the amount of the enzyme to be bonded is very small and the bonding ratio was the highest in the cases of FSM/18 and FSM/M20. In silica gel having a heterogeneous pore size (average pore size: 2.5 nm) shown as the comparative example, adsorption of the enzyme was hardly recognized. The fact that an enzyme molecules are present in the pores was confirmed by a reduction in an amount of N₂ adsorbed into the pores, using a N₂ adsorption measuring apparatus.

**Table 1**

| Material | FSM/8 | FSM/10 | FSM/14 | FSM/16 | FSM/18 | FSM/M20 | Silica gel |
|---|---|---|---|---|---|---|---|
| Pore size (nm) (Average) | 1.4 | 1.6 | 2.3 | 2.7 | 3.2 | 4.7 | 2.5 |
| Bonding ratio (%) | | | | | | | |
| Cytochrome c | 17 | 23 | 73 | 86 | 96 | 95 | 1 |
| Subtilisin | 13 | 12 | 45 | 58 | 61 | 57 | 2 |

### Example 8

To each of FSM/14, FSM/16, FSM/18 and FSM/M20 in which subtilisin had been immobilized, 90 µl of 50 mM Tris-HCl (pH8.0) was added and the solution was treated at 80°C for a predetermined time. To this solution, 10 µl of a solution of Boc-GLL-pNA (manufactured by BACH Co.) having a concentration of 4 mg/ml was added as a substrate and the reaction was performed at 37°C for 30 minutes. Then, the residual enzyme activity was measured. For comparison, 1 µg of subtilisin was added to 90 µl of 50 mM Tris-HCl (pH8.0) and the solution was treated at 80°C for a predetermined time. To this solution, 10 µl of a solution of Boc-GLL-pNA (manufactured by BACHEM Co.) having a concentration of 4 mg/ml was added as a substrate and the reaction was performed at 37°C for 30 minutes. Then, the residual enzyme activity was measured.

The results are summarized in Table 2. As is apparent from the results shown in this table, FSM/18 (pore size: 3.2 nm) having a pore size closed to a diameter (3.2 nm) of the enzyme (subtilisin) and FSM/M20 (pore size: 4.7 nm) showed high stabilization effect, whereas, FSM/14 and FSM/16 having a small pore size showed small stabilization effect.

**Table 2**

| | Residual activity after 30 min. | Residual activity after 90 min. |
|---|---|---|
| Subtilisin alone | ≤5% | 0% |
| FSM/14-subtilisin | 15% | ≤5% |
| FSM/16-subtilisin | 20% | 5% |
| FSM/18-subtilisin | 80% | 50% |
| FSM/M20-subtilisin | 85% | 60% |

### Example 9

As the organic solvent, aqueous solutions of dioxane or dimethylformamide having different concentrates were prepared. On the other hand, the organic solvent of each concentration was charged in a glass tube containing 3 mg of FSM/18 or FSM/M20 in which subtilisin had been immobilized, and then the glass tube was maintained at 30°C for 20 hours. Then, the supernatant was removed by centrifugation and the residue was washed with 1ml of deionized water. To each of immobilized FSM/18 and FSM/M20, 10 µl of a solution of Boc-GLL-pNA (manufactured by BACHEM Co.) having a concentration of 4 mg/ml was added as a substrate and the reaction was performed at 37°C for 30 minutes. Then, the enzyme activity was measured.

By using a sample obtained by adding subtilisin alone to the organic solvent of each concentration and similarly maintaining the solution at 30°C for 20 hours, the residual activity was measured by as described above. The subtilisin immobilized in FSM maintained 100% activity at the organic solvent concentration of not more than 80%, whereas, it had 90% or higher residual activity even at the organic solvent concentration of 100%. On the other hand, in the case of the enzyme alone, the enzyme activity was not remained at the organic solvent concentration of not less than 80%.

### Example 10

To each of FSM/18 and FSM/20 in which subtilisin had been immobilized, 90 µl of 50 mM Tris-HCl (pH8.0) containing 40% dimethylformamide and 10 µl of a solution of Boc-GLL-pNA (manufactured by BACHEM Co.) having a concentration of 4 mg/ml as a substrate were added and the reaction was performed at 37°C for 30 minutes. Then, the enzyme activity was measured. After the measurement, the supernatant was removed by centrifugation and the residue was washed with 1ml of deionized water. The above measurement was repeated. The measurement was continuously performed ten times, but decrease of the activity was not recognized.

### Example 11

To 3 mg of each of FSM/18 and FSM/20 in which subtilisin had been immobilized or 30 µg of subtilisin, were added 90 µl of 50 mM Tris-HCl (pH8.0) and 10 µl of pronase E (manufactured by Sigma Co.) as a non-specific protease and the reaction was performed at 30°C for 5 or 20 hours. After the completion of the reaction, 10 µl of a solution of Boc-GLL-pNA (manufactured by BACHEM Co.) having a concentration of 4 mg/ml as a non-specific substrate were added and the reaction was performed at 37°C for 30 minutes. Then, the residual enzyme activity was measured. The results are shown in Table 3. It became apparent that the enzyme immobilized in FSM exhibits strong resistance to a protease.

### Example 12

To 3 mg of FSM/20 in which subtilisin had been immobilized or 10 µg of subtilisin, has added 90 µl of a solution having a different concentration prepared by diluting dimethylformamide with deionized water. To the solution, 10 µl of a solution of Boc-GLL-pNA (manufactured by BACHEM Co.) having a concentration of 4 mg/ml prepared by dissolving in dimethylformamide was added as a specific substrate and the reaction was performed at 37°C for 30 minutes. Then, the enzyme activity was measured. The results are shown in Table 3.

It became apparent that the enzyme activity of the enzyme immobilized in FSM was remained to some extent even if the concentration of the organic solvent (dimethylformamide) becomes high.

**Table 3**

| Resistance to protease | | |
|---|---|---|
| | Residual activity (%) after 5 hours | Residual activity (%) after 20 hours |
| Subtilisin alone | 8 | 0 |
| FSM/18-subtilisin | 98 | 96 |
| FSM/M20-subtilisin | 97 | 95 |

### Example 13. Method for synthesis of kanemite mesoporous material FSM-R

### Synthesis of FSM/16-19

Cation exchange was performed by charging 5.0 g (0.028 mol) of delta-Na₂Si₂O₆ and 50 ml of deionized water in a 100 ml beaker and stirring at room temperature (about 25°C) for 3 hours. The aqueous solution was isolated by filtration to obtain delta-Na_{1.6}H_{0.4}Si₂O₅ as a precipitate. 50 ml of deionized water was poured into this precipitate and stirring was performed until a uniform solution was obtained, thereby to obtain a solution A. 3.0 g (0.082 mol) of hexadecyltrimethylammonium bromide (HDTMA-Br) and 50 ml of deionized water were charged in a 100 ml Erlenmeyer flask, followed by stirring. After the solution became completely transparent, 5.0 g (0.025 mol) of triisopropylbenzene (TIPB) was added and the solution was vigorously stirred for 10 minutes to obtain a solution B.

The above solution A was transferred to a 250 ml three-necked round bottom flask and the solution B was gradually added thereto with stirring vigorously. The solution was heated to 80°C and then continuously reacted at a predetermined temperature for 3 hours. The pH of the reaction solution was adjusted to 8.5±0.1 by using 2N hydrochloric acid, followed by continuous stirring for 3 hours. Immediately after the completion of the reaction, the solution was filtered, washed five times with 200 ml of deionized water and then filtered.

The product (white powder) was air-dried at 45°C for 24 hours, and calcined in an electric oven at 550°C for 6 hours to obtain about 3.5 g of a mesoporous material in which a template had been removed. The structure of the mesoporous material was confirmed by using a X-ray diffraction apparatus (Rigaku RAD-B) and the pore size, surface area and total pore volume of the mesoporous material were measured by using a N₂ absorb.

### Synthesis of FSM22-7

Cation exchange was performed by charging 5.0 g (0.028 mol) of delta-Na₂Si₂O₆ and 50 ml of deionized water in a 100 ml beaker and stirring at room temperature (about 25°C) for 3 hours. The resulting agueous solution was filtrated to obtain delta-Na_{1.6}H_{0.4}Si₂O₅ as a precipitate. 50 ml of deionized water was poured into this precipitate and stirring was performed until a uniform solution was obtained, thereby to obtain a solution A.

4.0 g (0.01 mol) of dococyltrimethylammonium chloride (DTMA-Cl) and 50 ml of deionized water were charged in a 100 ml Erlenmeyer flask, followed by stirring at 60°C. After the solution became completely transparent, 8.0 g (0.04 mol) of triisopropylbenzene (TIPB) was added and the solution was vigorously stirred for 10 minutes and this solution was maintained at 60°C to obtain a solution B. The above solution A was transferred to a 250 ml three-necked round bottom flask and the solution B was gradually added thereto with stirring vigorously. The solution was heated to 80°C and then continuously reacted at a fixed temperature for 3 hours.

The pH of the reaction solution was adjusted to 8.5±0.1 by using 2N hydrochloric acid, followed by continuous stirring for 3 hours. Immediately after the completion of the reaction, the solid product was collected by filtration, washed five times with 200 ml of deionised water and then filtered. The product (white powder) was air-dried at 45°C for 24 hours, and calcined in an electric oven at 550°C for 6 hours to obtain about 4.5 g of a mesoporous material in which a template had been removed. The structure of the mesoporous material was confirmed by using a X-ray diffraction apparatus (Rigaku RAD/B) and the pore size, surface area and total pore volume of the mesoporous material were measured by using a N₂ absorb.

### Example 14. Method for synthesis of water glass mesoporous materials MCM41/22 and MCM41/22

7.3 g (0.018 mol) of a chlorate surfactant (C22TMACl) of dococyltrimethylamine and 60 ml of deionized water were charged in an Erlenmeyer flask and, after completely dissolving at 60°C, 24 ml of an aqueous 10% (w/w) sulfuric acid solution was added to obtain a solution A. 10 g of water glass (SiO₂, 36 to 38%, Na₂O, 17-195) and 20 ml of deionized water were charged in a 100 ml beaker to obtain a solution B.

The solution B was gradually added to the solution A with stirring vigorously at 60 to 65°C. The pH of the reaction solution was adjusted to 8.5±0.1 by using 10% (w/w) sulfuric acid, followed by continuous stirring for 5-8 hours. Then, this mixed solution was transferred to a 250 ml autoclave and heated at 105°C for 2 to 5 days. The reaction solution was filtered under vacuum to obtain about 9 g of a crude product.

The crude product was air-dried at 45°C for 24 hours, and calcined in an electric oven at 550°C for 6 hours to obtain about 4.5 g of a mesoporous material in which a template had been removed. The structure of the mesoporous material was confirmed by using a X-ray diffraction apparatus (Rigaku RAD-B) and the pore size, surface area and total pore volume of the mesoporous material were measured by using a N₂ absorb.

The product produced by synthesizing by the above reaction, except for adding 4.9 g of triisopropylbenzene (TIPB) as an inflating agent to the dococyltrimethylammonium chloride solution, was designated as MCM41/M22.

Furthermore, the product produced by synthesizing by the above reaction, except for using hexadecyltrimethylammonium chloride [C16bH33n(CH₃)₃Cl] in place of dococyltrimethylammnoum chloride, was designated as MCM41/16.

### Example 15.

A solution of peroxidase (manufactured by Sigma Co.) derived from horseradish, as an enzyme, having a concentration of 5 mg/ml was prepared by using a 50 mM sodium acetate buffer solution (pH3 or 5) or a 50 mM Tris-hydrochloric acid buffer solution (pH3 or 5). To 200 mg of each of synthesized various mesoporous materials (FSM or MCM41), 5 ml of each of various enzyme solutions was added, followed by mild-mixing at 4°C for not less than 4 hours.

The supernatant was removed by centrifugation to recover the resulting porous material to which the enzyme had been bonded, and the porous material was washed three times with deionized water. In this case, the amount of the bonded enzyme was calculated by measuring absorbance at 403 nm of the original enzyme solution, the supernatant and washing solution.

The results are summarized in Table 4. As a result, it became apparent that FSM has a larger bonding capability of the peroxidase and the amount of the bonded enzyme is very small in the case of MCM41. It became apparent that FSM/M20 having a pore size of 4.7 among FSMs exhibits high adsorption capability. It also became apparent that the bonding ratio is higher under the acidic conditions.

**Table 4**

| Material Average pore size | FSM/10 1.6 | FSM/16 2.7 | FSM/18 3.2 | FSM/20 4.7 | MCM41/16 2.7 | MCM41/22 4.3 | MCM41/M22 5.8 |
|---|---|---|---|---|---|---|---|
| Bonding ratio (%) | | | | | | | |
| pH3 Buffer solution | 5 | 23 | 53 | 76 | 17 | 25 | 30 |
| pH5 Buffer solution | 4 | 28 | 45 | 68 | 12 | 20 | 24 |
| pH7 Buffer solution | 2 | 10 | 28 | 26 | 8 | 6 | 8 |
| pH9 Buffer solution | 5 | 11 | 12 | 11 | 2 | 1 | 1 |

### Example 16. Oxidation activity of peroxidase-immobilized mesoporous material in toluene

1,2-diaminobenzene was dissolved in 10 ml of toluene so that the concentration becomes 20 mM. To this solution, 2 ml of a solution of t-butylhydroxy peroxide prepared by dissolving in a decane solution so that the concentration becomes 0.05 M was added. To the solution, each of various peroxidase-immobilized mesoporous materials (FSM/18 made in Example 1, FSM/M20 made in Example 2 and MCM41/M22 made in Example 14) was added in the amount of 0.1 mg in terms of the enzyme and the reaction was performed. Then, the absorbance at 470 nm of 1,2-dinitrobenzene produced by the oxidation reaction at 38°C was measured.

For a comparison test, peroxidase itself and an enzyme coated with glycolipid so that the activity can be exerted in the organic solvent were prepared according to the method of GOTO et al. (Biotechnology Technique, Vol. 11, No. 6, 375-378, 1997) and the above oxidation reaction was performed by using each enzyme in the amount of 0.1 mg in terms of the enzyme (peroxidase). Then, an increase in absorbance at 470 nm was measured.

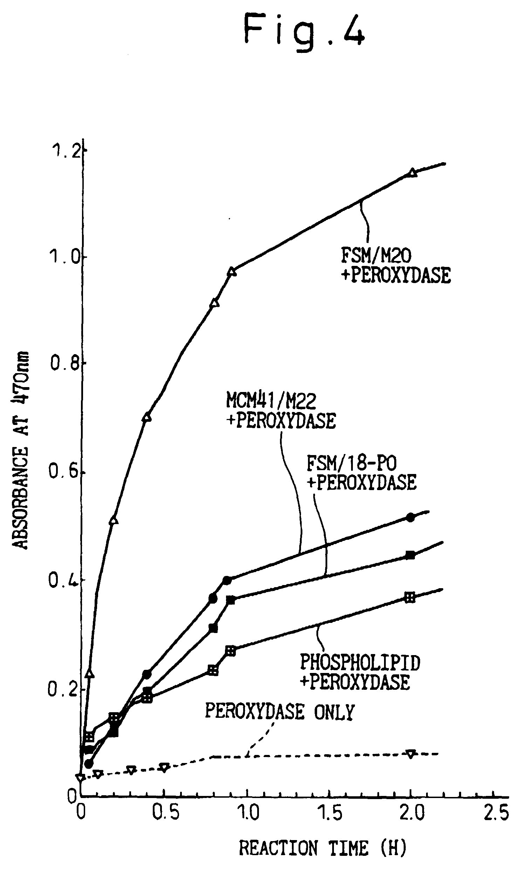

As a result, as shown in Fig. 4, oxidation activity was hardly exhibited in toluene in case of using only the enzyme. The mesoporous material in which peroxidase had been immobilized showed activity better than that of the enzyme coated with glycolapid, which is considered to exhibit most excellent activity among the organic solvents. In the case of one immobulized in FSM/M20, the activity was particularly high.

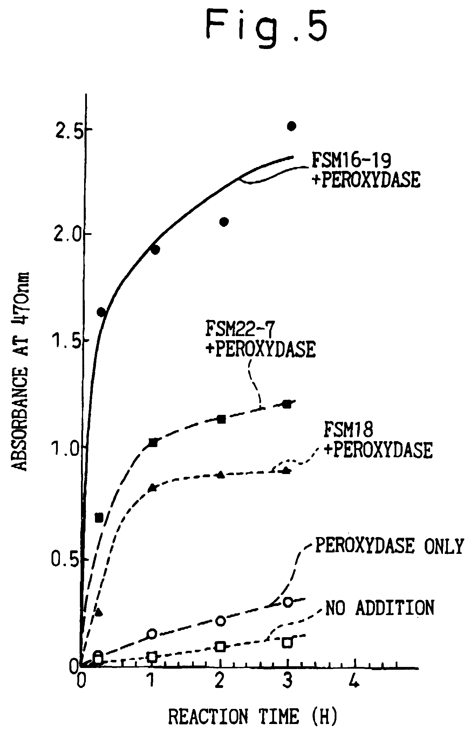

In the same manner as described above, 20 ml of toluene was used as the solvent and the concentration of 1,2-diaminobenzene was adjusted to 50 mM and, furthermore, the amount of the peroxidase enzyme in the used mesoporous material was set to 0.3 mg. As the immobilized enzyme, FSM16-19 (pore size: 5.0 nm, amount of enzyme immobilized: 3.8%) and FSM22-7 (pore size: 6.5 pm, mount of enzyme immobilized: 2.8%) made in Example 3 and FSM18 (pore size: 3.2 nm, amount of enzyme immobilized: 9.0%) made in Example 1 were used. The reaction was also performed in the case of using only peroxidase or adding no enzyme. The absorbance at 470 nm (obtained by subtracting a back value of a FSM solid acid as a carrier) of the reaction solution was taken as an index of a reaction conversion rate. The results are shown in Fig. 5.

The size of peroxidase is about 4.6 mm. In the case of FSM16-19 (pore size: 5.0 nm) having a pore size closed to the size of peroxidase, the reactivity was the highest. In the case of FSM22-7 (pore size: 6.5 nm) having a pore size larger than the size of the enzyme and FSM18 (pore size: 3.2 nm) having a pore size smaller than the size of the enzyme, the reactivity was low.

### Example 17. Liquid phase adsorption test

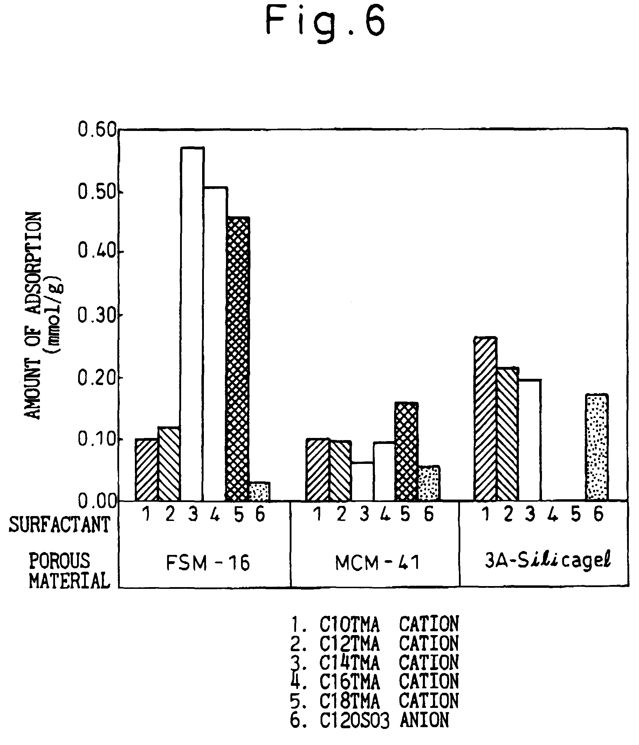

To each of various absorbent materials 0.4 g each of 0.1 M surfactant solutions was added, followed by stirring in a hot water bath at 70°C for 5 hours. After the completion of the reaction, the reaction solution was suction-filtered, washed with deionized water and then allowed to stand in a drier at 45°C overnight. The amount of the surfactant adsorbed was determined by the measurement of by thermogravimetric defferential thermal analysis (TG/DTA). TG/DTA was performed at a heating rate of 20°C /min. from the test temperature to 100°C and, after absorbed water was evaporated by maintaining for 25 min., TG/DTA was performed at a heating rate of 20°C /min. to 900°C. It was estimated that the amount of the surfactant adsorbed is a weight loss from 100 to 900°C. In that case, the calculation was performed by using the molecular weight in a state of a cation or an anion of each surfactant. The results are shown in Table 5 and Fig. 6.

FSM-16 shows the amount of the cationic surfactant adsorbed, which is 5 times high as MCM-41. In the case of the anionic surfactant, significant difference was not recognized.

**Table 5**

| | | FSM-16 | MCM-41 | 3A-Silica gel |
|---|---|---|---|---|
| Cation | C10TMA | 0.10 | 0.10 | 0.26 |
| | C12TMA | 0.12 | 0.10 | 0.21 |
| | C14TMA | 0.57 | 0.06 | 0.19 |
| | C16TMA | 0.51 | 0.09 | 0.02 |
| | C18TMA | 0.46 | 0.16 | 0.00 |
| Anion | C12OSO | 0.03 | 0.06 | 0.17 |
| Pore size (nm) | | 2.7 | 2.6 | 2.5 |
| Specific surface area (m²/g) | | 912 | 910 | 677 |
| External surface area (m²/g) | | 201 | 87 | |
| Ratio (%) | | 22.1 | 9.5 | |

## Claims

1. An immobilized enzyme comprising
- a structural unit (1) having structural stability; and
- an enzyme having an active unit (2), the enzyme or active unit (2) being immobilized to said structural unit (1).

2. The immobilized enzyme according to claim 1, wherein the structural unit (1) is a porous substance having homogeneous pores, preferably wherein the porous substance has a pore size distribution whose half-height width is not more than 60 %, based on the center pore diameter.

3. The immobilized enzyme according to claim 1 or claim 2, wherein the pore size (diameter) of said porous substance of the structural unit (1) is almost the same as that of the enzyme to be immobilized or the active unit (2) of the enzyme, preferably wherein the pore size (diameter) of said porous substance of the structural unit (1) is larger than 2 nm.

4. The immobilized enzyme according to any of the claims 1 to 3, wherein the structural unit (1) is an inorganic substance, preferably wherein the inorganic substance is a silicon-containing compound, more preferably wherein the silicon-containing compound is a mesoporous silica porous material to be formed via a layered silicate, even more preferably wherein said mesoporous silica porous material is FSM.

5. The immobilized enzyme according to any of the claims 1 to 3, wherein the structural unit (1) is an organic substance, preferably wherein the organic substance is synthesized from at least one monomer selected from the group consisting of acrylic acid, methyl (meth)acrylic acid, ethyl (meth)acrylic acid, methyl (meth)acrylate, ethyl (meth)acrylate, hydroxyethyl (meth)acrylate, hydroxypropyl (meth)acrylate, styrene, divinylbenzene, acrylamide and 4-vinylpyridine.

6. The immobilized enzyme according to any of the claims 1 to 5, wherein the surface of the structural unit (1) is anionic or wherein the surface of the structural unit (1) is hydrophobic.

7. The immobilized enzyme according to any of the claims 1 to 6, wherein the enzyme or the active unit (2) is immobilized to the structural unit (1) by a van der Waals force.

8. The immobilized enzyme according to any of the claims 1 to 7, said structural unit (1) further comprising an anchor unit (3) for immobilizing the enzyme or the active unit (2) of the enzyme to the structural unit (1).

9. The immobilized enzyme according to claim 8, wherein the anchor unit (3) has at least one functional group selected from the group consisting of hydroxyl group, amide group, amino group, pyridine group, urea group, urethane group, carboxyl group, phenol group, azo group, silane derivative and aminoalkylene group.

10. The immobilized enzyme according to any of the claims 3 to 9, wherein the pore size (diameter) of said porous substance of the structural unit (1) is smaller than 30 nm, preferably smaller than 10 nm.

11. The immobilized enzyme according to any of the claims 1 to 10, wherein the enzyme is at least one selected from the group consisting of oxidases, reductases, hydrolases, lipases and isomerases, preferably wherein the enzyme is at least one selected from the group consisting of peroxidases, subtilisin and lipases.

12. The immobilized enzyme according to any of the claims 1 to 10, wherein the enzyme comprises two or more different kinds of enzymes having almost the same molecular weights and/or almost the same particle sizes.

13. The immobilized enzyme according to claim 1, wherein the structural unit (1) is a porous substance having two or more peaks of the pore size distribution.

14. The immobilized enzyme according to claim 13, wherein the porous substance has at least one pore size distribution whose half-height width is not more than 60 %, based on the center pore diameter.

15. The immobilized enzyme according to claim 13 or claim 14, wherein the enzyme comprises comprises two or more different kinds of enzymes having different molecular weights and/or different particle sizes.
